# EUROPÄISCHE PATENTSCHRIFT

(11) **EP 0 505 724 B1**
(45) Veröffentlichungstag und Bekanntmachung des Hinweises auf die Patenterteilung: **11.09.1996**
(21) Anmeldenummer: 92102728.0
(22) Anmeldetag: 19.02.1992
(51) Int. Cl.: A45D 34/04

(54) **Vorrichtung zum Auftragen von Flüssigkeiten auf Körperteile**
Device for applying liquids on parts of the body
Dispositif pour appliquer des liquides sur des parties du corps

(30) Priorität: 23.03.1991 DE 9103587 U
(43) Veröffentlichungstag der Anmeldung: 30.09.1992
(73) Patentinhaber: Woebke, Klaus, D-89537 Giengen (DE)
(72) Erfinder: Woebke, Klaus, D-89537 Giengen (DE)
(74) Vertreter: Lorenz, Werner, Dipl.-Ing.

(56) Entgegenhaltungen:
- CH-A- 370 525
- CH-A- 641 656
- DE-C- 827 701
- FR-A- 1 355 728
- FR-A- 2 601 583
- FR-A- 2 619 308
- US-A- 4 207 909

## Beschreibung

Die vorliegende Erfindung betrifft eine Vorrichtung zum Auftragen von Flüssigkeiten auf Körperteile, insbesondere von Körperlotion, Sonnenschutzcreme, Dusch- und Bademittel oder cremigen oder flüssigen Arzneimitteln und dergleichen mit einem Behälter, in dem sich die aufzutragende Flüssigkeit befindet, wobei der kreis- oder ovalförmige Behälter mit einer Vielzahl von Flüssigkeitsauftragskugeln versehen ist, die in Lagerbohrungen einer eine Wand des Behälters bildenden Abdichteinheit derart drehbar angeordnet sind, daß jeweils ein Teil der Kugeloberflächen im Inneren des Behälters in Kontakt mit der aufzutragenden Flüssigkeit ist und ein anderer Teil aus der Wand des Behälters herausragt, und wobei der Behälter oben offen ist und auf dieser Seite mit einem Deckel abschließbar ist.

Beim Auftragen von Körperlotion, Sonnenschutzcremes und dergleichen benötigt man bisher die Hände, was oft als unangenehm bzw. klebrig empfunden wird. Zum Auftragen der Flüssigkeiten auf schwer zugängliche Stellen, z.B. auf den Rücken oder bei kranken bzw. behinderten Personen ist die Hilfe einer dritten Person notwendig.

Bekannt sind z.B. Stielbadebürsten, wobei jedoch eine auf die Borsten aufgebrachte Flüssigkeit beim Auftragen schnell nach unten abfließt und somit verloren geht.

Bei den bekannten Deorollern handelt es sich um meist flaschenförmige Behälter, die mit einer Drehkugel versehen sind. Dabei wird durch ein Senkrechtstellen des Behälters mit der Drehkugel nach unten diese mit der im Behälter befindlichen Flüssigkeit benetzt. Beim Abrollen der Drehkugel trägt diese dann die Flüssigkeit vom Behälterinneren nach außen. Für ein Auftragen von Flüssigkeiten in größerem Umfang sind Deoroller jedoch nicht geeignet.

Aus der CH-A-641 656 ist ein ovaler Behälter bekannt, in dem zwei Kugeln, die unter einer Federvorspannung stehen, teilweise aus dem Behälter herausragen. Zweck dieses Behälters ist es, flüssige Seife aus dem Behälterinneren auszugeben. Die flüssige Seife wird dabei oben in den Behälter, der auf dieser Seite offen ist bzw. mit einer Öffnung versehen ist, eingefüllt. Mit einem Deckel ist die Behälteröffnung abschließbar.

Diese bekannte Vorrichtung ist jedoch lediglich zur Ausgabe von Flüssigkeiten, wie eben flüssiger Seife, vorgesehen, wobei diese Ausgabe auch nur in einem beschränkten Umfange möglich ist. Zum großflächigen Auftragen auf den Körper, insbesondere an schwer Zugänglichen Stellen, ist die vorbekannte Vorrichtung nicht geeignet.

In der FR-A-2 619 308 ist ein Handmassagegerät mit einem elektrischen Antrieb offenbart. Das Gerät selbst ist als eine einzige Einheit ausgebildet und nicht zum Auftragen von Flüssigkeiten auf Körperteile, insbesondere von Körperlotion, Sonnenschutzcreme, Dusch- und Bademittel oder cremigen oder flüssigen Arzneimitteln vorgesehen.

Aus der US-A-4 207 909 ist eine Vorrichtung zum Auftragen von Flüssigkeit bekannt, die in Form eines Armes ausgebildet ist. Die Flüssigkeit soll hierbei auf die Kopfhaut einer Person aufgebracht werden. Hierzu sind Flüssigkeitsauftragskugeln in den einer menschlichen Hand nachgebildeten Fingerspitzen vorgesehen.

Die Kugeln sind in den Fingern jeweils einzeln angeordnet, wobei die einzelnen Finger nach hinten offen und dort durch einen gemeinsamen Füllarm zum Zuführen einer Flüssigkeit miteinander verbunden.

Der vorliegenden Erfindung liegt die Aufgabe zugrunde, eine Vorrichtung zum Auftragen von Flüssigkeiten auf Körperteile zu schaffen, durch die das Auftragen einfach und schnell und im Bedarfsfalle ohne Zuhilfenahme der Hände erfolgen kann, und zwar insbesondere auf größere Flächen.

Erfindungsgemäß wird diese Aufgabe dadurch gelöst, daß die Flüssigkeitsauftragskugeln in mehreren Kreisringen auf der Unterseite des Behälters, welche die Abdichteinheit beinhaltet, angeordnet sind, wobei der Behälter durch Stege in mehrere gegeneinander abgedichtete Kammern unterteilt ist und mindestens eine Flüssigkeitsauftragskugel in Kontakt mit dem Inneren einer Kammer ist, und daß an dem Deckel ein Stiel angeordnet ist.

Auf diese Weise ist eine einfache und schnelle Auftragung auf eine große Fläche mit gleichmäßiger Verteilung, gegebenenfalls ohne Zuhilfenahme der eigenen oder fremder Hände möglich. Die Hände werden nicht schmutzig bzw. sind nicht von Creme oder ähnlichem verschmiert. Außerdem bleibt auf diese Weise auch die Creme, Lotion oder dergleichen sauber. Durch die erfindungsgemäße Ausgestaltung wirken die Flüssigkeitsauftragskugeln auch auf sehr angenehme Weise massageartig.

Durch die Unterteilung des Behälters durch Stege in mehrere gegeneinander abgedichtete Kammern ist eine gute und gleichmäßige Verteilung der Flüssigkeit in dem Behälter gewährleistet. Insbesondere bei einem Senkrechthalten der erfindungsgemäßen Vorrichtung sammelt sich im teilentleerten Zustand die Flüssigkeit nicht nur im unteren Teil des Behälters an, womit die oberen Kugeln funktionslos wären.

Die Flüssigkeitsauftragskugeln sind platzsparend in mehreren Kreisringen auf der Unterseite des Behälters, der die Abdichteinheit beinhaltet, angeordnet, wodurch auch eine einfache Ausgestaltung der erfindungsgemäßen Vorrichtung erreicht ist.

Da immer eine Flüssigkeitsauftragskugel in Kontakt mit dem Inneren einer Kammer ist, ist gewährleistet, daß permanent die in den Behälter eingefüllte Flüssigkeit auf die Haut aufgetragen wird.

Eine besonders einfache Handhabung der erfindungsgemäßen Vorrichtung ist durch den an dem Deckel des Behälters angebrachten Stiel gewährleistet.

Von Vorteil ist es, wenn die Flüssigkeitsauftragskugeln von verschiedenen Kreisringen in radialer Richtung versetzt zueinander angeordnet sind.

Eine derartige versetzte Anordnung bedeutet eine bessere und gleichmäßigere Verteilung der Flüssigkeit, d.h. wenig Leerstellen.

In vorteilhafter Ausgestaltung der Erfindung können der Behälter und der Deckel durch in beiden Teilen angeordnete Gewinde miteinander verbunden sein, wodurch einfache und schnell lösbare Verbindungen geschaffen sind.

Bei einer weiteren vorteilhaften Ausgestaltung können der Behälter und der Deckel durch einen Paßsitz miteinander verbunden sein, wodurch die Möglichkeit einer einfachen und schnell lösbaren Verbindung noch weiter verbessert ist.

Der an dem Deckel des Behälters angebrachte Stiel kann vorteilhafterweise auswechselbar mit dem Deckel verbunden sein.

Dadurch sind die Anwendungsmöglichkeiten erweitert. So läßt sich die erfindungsgemäße Vorrichtung beispielsweise auch ohne störenden Stiel an den Beinen, am Bauch usw. verwenden.

Bei einer weiteren Ausgestaltung kann der Stiel über eine Gewindeverschraubung oder Steckverbindung mit dem Deckel verbunden sein, was eine sehr einfache und leicht lösbare Art der Verbindung darstellt.

Der Behälter kann auf der Seite, auf der die Flüssigkeitsauftragskugeln aus ihm herausragen, mit einer Schutzkappe abdeckbar sein.

Durch eine derartige Schutzkappe wird eine Verschmutzung der Person und/oder von Kleidern bei Nichtgebrauch durch eine evtl. austretende Flüssigkeit oder durch noch feuchte Kugeln verhindert.

Sehr von Vorteil ist es, wenn die Schutzkappe aus einem elastischen Kunststoff besteht und mit Paßsitz auf den Behälter aufgeschoben ist.

Auf diese Weise ist ein einfaches und schnelles Aufbringen der Schutzkappe möglich.

Vorteilhaft ist es, wenn die Kammern eine derartige Größe besitzen, daß jeweils zwei Flüssigkeitsauftragskugeln in eine Kammer ragen. Dies hat sich in der Praxis als besonders geeignete Anordnung herausgestellt.

Nachfolgend ist anhand der Zeichnung schematisch ein Ausführungsbeispiel der Erfindung dargestellt.

Es zeigt:
- Fig. 1: eine Seitenansicht eines mit Deckel versehenen Behälters, wobei der Deckel mit einem Stiel versehen ist,
- Fig. 2: eine Draufsicht auf den Behälter ohne Deckel,
- Fig. 3: eine auseinandergezogene Ansicht, teilweise im Schnitt, des Deckels mit dem Behälter.

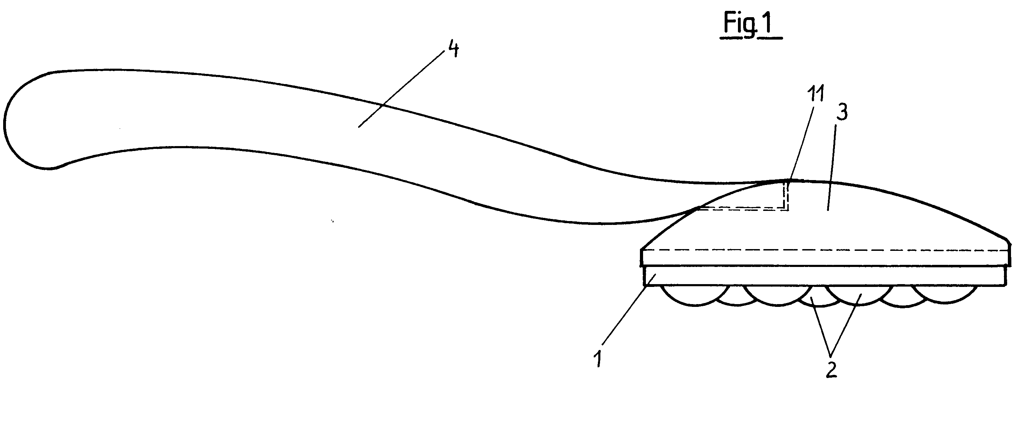

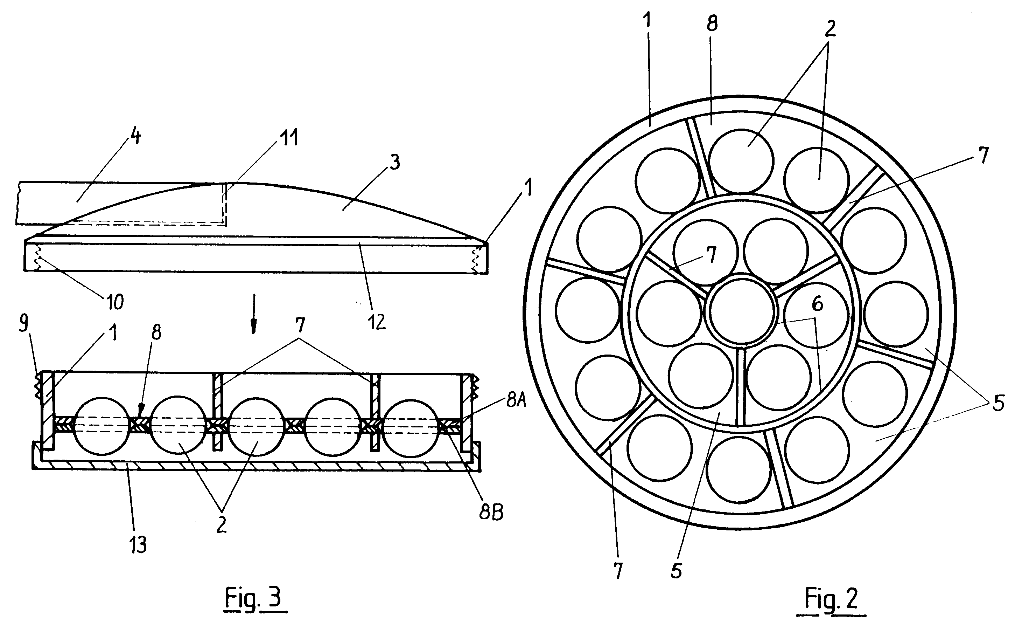

Die Fig. 1 zeigt einen Behälter 1, auf dessen Unterseite eine Vielzahl von Flüssigkeitsauftragskugeln 2 angeordnet ist. Die Flüssigkeitsauftragskugeln 2 sind dabei in (Fig. 3) Lagerbohrungen drehbar angeordnet, wobei der Durchmesser der Lagerbohrungen kleiner ist als der Durchmesser der Flüssigkeitsauftragskugeln 2 (siehe nachfolgende Erläuterung).

Auf der oberen Seite des Behälters 1 ist dieser mit einem Deckel 3 verschlossen. In eine Gewindebohrung 11 oder eine Steckbohrung des Deckels 3 ist ein geschwungener Stiel 4 eingeschraubt oder eingesteckt.

In der Fig. 2 ist der runde Behälter 1 ohne Deckel dargestellt. In seinem Inneren ist eine Vielzahl von Flüssigkeitsauftragskugeln 2 in einzelnen Kammern 5 angeordnet. Die einzelnen Kammern 5 sind durch Kreisringe 6 und mehrere Stege 7 gebildet. Jeweils zwei Flüssigkeitsauftragskugeln 2 befinden sich in einer Kammer 5. Selbstverständlich ist diese Anzahl nur beispielsweise genannt. Im Bedarfsfalle kann die Aufteilung auch anders sein.

Aus der Fig. 3 ist ein Schnitt durch den Behälter 1 ersichtlich, in dem eine Vielzahl von Flüssigkeitsauftragskugeln 2 zwischen Stegen 7 angeordnet sind. Durch den im wesentlichen mittleren Bereich des Behälters 1 erstreckt sich eine Abdichteinheit 8, die aus zwei miteinander verbundenen Lagerplatten 8A und 8B besteht.

Der gesamte untere Bereich des Behälters 1, d.h. die Seite, auf der die Flüssigkeitsauftragskugeln aus dem Behälter 1 herausragen, ist von einer Schutzkappe 13 abgedeckt, die zum Gebrauch entfernt wird.

An dem Behälter 1 ist im oberen Bereich ein Gewinde 9 zum Aufschrauben des Deckels 3 angeordnet. Das Gewinde 9 wirkt hierzu mit einem Innengewinde 10 zusammen, welches im unteren Bereich des Deckels 3 in dessen nach unten ragenden Rand in die Innenwand eingeformt ist.

Der obere Bereich des Deckels 3, d.h. die vom Behälter 1 abgewandte Seite, ist kuppelförmig ausgebildet, wobei jedoch unter der Kuppel eine Abdichtwand 12 angeordnet ist, die im geschlossenen Zustand den Behälter 1 nach oben und auch die einzelnen Kammern 5 durch die sich bis zur Behälteroberseite erstreckenden Stege 7 und Kreisringe 6 gegeneinander abdichtet. Hierzu liegt die Abdichtwand 12 dichtend auf der Umfangswand des Behälters 1 auf, was durch das Aufschrauben des Deckels 3 auf den Behälter 1 erfolgt. In dem Kuppelbereich ist der Stiel 4 in der Gewinde- oder Steckbohrung angeordnet.

Beide Lagerplatten 8A und 8B besitzen Lagerbohrungen, die in ihren Durchmessern geringfügig kleiner sind als die Durchmesser der Flüssigkeitsauftragskugeln 2. Die Lagerbohrungen sind jedoch schräg bzw. derart konisch, daß sie im Bereich ihres größten Durchmessers größer sind als die Durchmesser der Flüssigkeitsaufnahmekugeln 2. Die beiden Lagerplatten 8A und 8B sind dabei so übereinander gesetzt, daß die größeren Durchmesser einander zugekehrt sind. Auf diese Weise wird - wie an sich bekannt - erreicht, daß die Flüssigkeitsaufnahmekugeln mit Spiel zwischen den beiden Lagerplatten 8A und 8B gehalten werden, d.h. drehbar sind, jedoch nicht durchfallen können.

Für einen Gebrauch der Vorrichtung wird Deckel 1 abgenommen und die gewünschte Flüssigkeit in den Behälter 1 eingefüllt, und zwar genauer gesagt in die Kammern 5. Anschließend wird der Deckel 3 wieder auf den Behälter geschraubt und ggf. auch der Stiel 4 in die Gewindebohrung 11 eingeschraubt. Nach Abnahme der Schutzkappe 13 kann die Vorrichtung auf das Körperteil aufgesetzt werden, auf dem die Flüssigkeit verteilt werden soll. Durch die Abrollbewegung der Flüssigkeitsaufnahmekugeln 2 gelangt damit die Flüssigkeit aus den Kammern 5 über die Kugeloberflächen nach außen, wo sie auf der gewünschten Fläche bzw. dem gewünschten Körperteil aufgetragen und während der Bewegung der Vorrichtung auch weitgehend gleichmäßig darauf verteilt wird. Gleichzeitig stellt sich durch die Bewegung der Flüssigkeitsauftragskugeln auch noch ein Massageeffekt ein. Durch die Kammern 5 ist auch bei einer senkrechten Gebrauchshaltung der Vorrichtung gewährleistet, daß fast bis zur völligen Entleerung der Flüssigkeit nahezu alle Flüssigkeitsauftragskugeln 2 in Funktion sind.

## Patentansprüche

1. Vorrichtung zum Auftragen von Flüssigkeiten auf Körperteile, insbesondere von Körperlotion, Sonnenschutzcreme, Dusch- und Bademittel oder cremigen oder flüssigen Arzneimitteln und dergleichen mit einem Behälter (1), in dem sich die aufzutragende Flüssigkeit befindet, wobei der kreis- oder ovalförmige Behälter (1) mit einer Vielzahl von Flüssigkeitsauftragskugeln (2) versehen ist, die in Lagerbohrungen einer eine Wand des Behälters (1) bildenden Abdichteinheit (8) derart drehbar angeordnet sind, daß jeweils ein Teil der Kugeloberflächen im Inneren des Behälters (1) in Kontakt mit der aufzutragenden Flüssigkeit ist und ein anderer Teil aus der Wand des Behälters (1) herausragt, und wobei der Behälter (1) oben offen ist und auf dieser Seite mit einem Deckel (3) abschließbar ist,
**dadurch gekennzeichnet,** daß die Flüssigkeitsauftragskugeln (2) in mehreren Kreisringen (6) auf der Unterseite des Behälters (1), welche die Abdichteinheit (8) beinhaltet, angeordnet sind, wobei der Behälter (1) durch Stege (7) in mehrere gegeneinander abgedichtete Kammern (5) unterteilt ist und mindestens eine Flüssigkeitsauftragskugel (2) in Kontakt mit dem Inneren einer Kammer (5) ist, und daß an dem Deckel (3) ein Stiel (4) angeordnet ist.

2. Vorrichtung nach Anspruch 1,
**dadurch gekennzeichnet,** daß die Flüssigkeitsauftragskugeln (2) von verschiedenen Kreisringen (6) in radialer Richtung versetzt zueinander angeordnet sind.

3. Vorrichtung nach Anspruch 1 oder 2,
**dadurch gekennzeichnet,** daß der Behälter (1) und der Deckel (3) durch in beiden Teilen angeordnete Gewinde (9,10) durch einen Paßsitz miteinander verbunden sind.

4. Vorrichtung nach einem der Ansprüche 1 bis 3,
**dadurch gekennzeichnet,** daß der Stiel (4) auswechselbar mit dem Deckel (3) verbunden ist.

5. Vorrichtung nach einem der Ansprüche 1 bis 4,
**dadurch gekennzeichnet,** daß der Behälter(1), auf der Seite, auf der die Flüssigkeitsauftragskugeln (2) aus ihm herausragen, mit einer Schutzkappe (13) abdeckbar ist.

6. Vorrichtung nach einem der Ansprüche 1 bis 5,
**dadurch gekennzeichnet,** daß die Kammern (5) eine derartige Größe besitzen, daß jeweils zwei Flüssigkeitsauftragskugeln (2) in eine Kammer (5) ragen.

## Claims

1. Device for applying liquids to parts of the body, in particular body lotion, sun cream, shower and bath preparations or creamy or liquid medicaments and the like, comprising a container (1) in which the liquid to be applied is situated, the circular or oval container (1) being provided with a plurality of liquid-applicator balls (2) rotatably arranged in bearing bores in a sealed unit (8) forming one wall of the container (1) in such a manner that part of the ball surfaces is in contact with the liquid to be applied in the interior of the container (1) and another part projects out from the wall of the container (1), wherein the container (1) is open at the top and can be closed by a lid (3) at this side, characterised in that the liquid-applicator balls (2) are arranged in a plurality of circular rings (6) on the underside of the container (1) containing the sealed unit (8), the container (1) being divided into a plurality of chambers (5) sealed off from one another by means of partitions (7) and at least one liquid-applicator ball (2) being in contact with the interior of one chamber (5), and that a handle (4) is arranged on the lid (3).

2. Device according to claim 1, characterised in that the liquid-applicator balls (2) of different circular rings (6) are arranged so that they are offset from one another in the radial direction.

3. Device according to claim 1 or claim 2, characterised in that the container (1) and the lid (3) are connected together with a snug fit by means of threads (9, 10) arranged in both parts.

4. Device according to one of claims 1 to 3, characterised in that the handle (4) is replaceably connected to the lid (3).

5. Device according to one of claims 1 to 4, characterized in that the container (1) can be covered with a protective cap (13) at the side at which the liquid-applicator balls (2) project therefrom.

6. Device according to one of claims 1 to 5, characterized in that the chambers (5) have dimensions such that two liquid-applicator balls (2) project into each chamber (5).

## Revendications

1. Dispositif pour appliquer des liquides sur des parties du corps, notamment des lotions corporelles, crèmes de protection solaire, des substances pour la douche ou le bain, ou des produits pharmaceutiques à l'état de crèmes ou de liquides ou similaires, comportant un réservoir (1) dans lequel est disposé le fluide à appliquer, dans lequel le réservoir de forme circulaire ou ovale (1) est équipé d'une série de billes applicatrices, qui sont logées dans des évidements d'une paroi du réservoir (1) formant une unité étanche (8), ces billes étant rotatives de telle manière qu'une partie de la surface périphérique des billes soit en contact avec le fluide à appliquer, à l'intérieur du réservoir (1) et une autre partie soit proéminente vers l'extérieur de la paroi du réservoir (1) et dans lequel, le réservoir (1) est ouvert à sa partie supérieure et peut être fermé au moyen d'un couvercle (3),
**caractérisé en ce que** les billes applicatrices de fluide (2) sont disposées selon plusieurs cercles concentriques (6) sur la face inférieure du réservoir (1) qui comporte l'unité étanche (8), dans lequel le réservoir (1) est subdivisé par des cloisons (7) en plusieurs chambres (5) étanches les unes par rapport aux autres, et dans lequel au moins une bille applicatrice de fluide (2) est en contact avec l'intérieur d'une chambre (5) et en ce que le couvercle (3) est pourvu d'un manche (4).

2. Dispositif selon la revendication 1,
**caractérisé en ce que** les billes applicatrices de fluide (2) sont disposées sur divers cercles concentriques (6), décalées selon une direction radiale.

3. Dispositif selon la revendication 1 ou 2,
**caractérisé en ce que** le réservoir (1) et le couvercle (3) sont liés par un assemblage comportant des filetages (9, 10) ménagés sur chacune de ces pièces.

4. Dispositif selon la revendication 1 à 3,
**caractérisé en ce que** le manche (4) est lié de façon interchangeable au couvercle (3).

5. Dispositif selon la revendication 1 à 4,
**caractérisé en ce que** le réservoir (1) est pourvu d'un capuchon de protection (13) agencé pour recouvrir les billes d'applications de fluide se projetant hors de ce réservoir.

6. Dispositif selon la revendication 1 à 5,
**caractérisé en ce que** les chambres (5) ont une dimension telle que deux billes applicatrices de fluide (2) pénètrent dans une même chambre (5).
